# EUROPÄISCHE PATENTSCHRIFT

(11) **EP 0 496 306 B1**
(45) Veröffentlichungstag und Bekanntmachung des Hinweises auf die Patenterteilung: **13.09.1995**
(21) Anmeldenummer: 92100798.5
(22) Anmeldetag: 18.01.1992
(51) Int. Cl.: C07D 487/04, C07D 403/06, C07D 223/20

(54) **Verfahren zur Herstellung von 3-Amino-9,13b-dihydro-1H-dibenz-[c,f]imidazol[1,5-a]azepin-hydrochlorid**
Process for the preparation of 3-amino-9,13b-dihydro-1H-dibenz-[c,f]imidazo[1,5-a]azepine hydrochloride
Procédé pour la préparation du chlorhydrate de la 3-amino-9,13-b-dihydro-1H-dibenz-[c,f]imidazo[1,5-a]azépine

(30) Priorität: 25.01.1991 DE 4102148
(43) Veröffentlichungstag der Anmeldung: 29.07.1992
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Schneider, Heinrich, Dr., W-6507 Ingelheim/Rhein (DE)

(56) Entgegenhaltungen:
- EP-A- 0 035 749
- ORGANIC SYNTHESES Bd. 61, 1983, JOHN WILEY & SONS, NEW YORK, U.S. Seiten 35 - 38; D. MARTIN , M. BAUER: 'Cyanic acid esters from phenols: phenyl cyanate'

## Beschreibung

Die Erfindung betrifft ein Verfahren zur Herstellung von 3-Amino-9,13b-dihydro-1-H-dibenz[c,f]imidazo[1,5-a]azepin-hydrochlorid.

Diese Verbindung gehört zur Gruppe der 2-Aminoimidazoline und stellt eine therapeutisch wirksame Substanz dar, die sich vor allem durch ihre antiallergische und antihistaminerge Wirkung auszeichnet (EP 35749).

2-Aminoimidazoline sind in großer Zahl bekannt. Zu ihrer Synthese werden Diamine mit Ringschlußreagenzien wie O-Alkylisoharnstoffen und S-Alkylisothioharnstoffen, insbesondere N-acylierten bzw. N-carbalkoxylierten, umgesetzt. Dieser Syntheseweg führt jedoch über viele Stufen und ist mit aufwendigen Reinigungsschritten verbunden. Er umfaßt die Acylierung bzw. Alkoxylierung der Alkyliso(thio)harnstoffe, die Kondensation mit dem Diamin, die Ringschlußreaktion, sowie die Verseifung des Acylrestes.

Ein weiterer Nachteil ist die bei der Verwendung von S-Alkylisothioharnstoffen auftretende Emission äquimolarer Mengen von Alkylmercaptanen.

Ähnliche Probleme bringt die Ringschlußreaktion durch Umsetzung mit Alkalicyanaten mit sich.

Demgegenüber gelingt die Ringschlußreaktion zu 2-Aminoimidazolinen mit Bromcyan in einem einzigen Reaktionsschnitt.

Aus der EP 35749 ist ein solches Verfahren zur Synthese des 3-Amino-9,13b-dihydro-1H-dibenz[c,f]imidazo[1,5-a]azepin-hydrochlorids bekannt. Hier werden die Reaktionsschritte wie folgt beschrieben:
6-Chlor-11H-dibenz[b,e]azepin wird durch Reaktion mit Natriumcyanid, Reduktion des entstandenen Nitrils mit Lithiumaluminiumhydrid und Reaktion mit Bromcyan zu der entsprechenden 2-Aminoimidazolinbase umgesetzt, die schließlich als Hydrochlorid aus einer methanolischen Suspension ausgefällt und isoliert wird. Die Ausbeute beträgt 41,8 %.

Im Rahmen einer technisch durchzuführenden Synthese ist es jedoch nicht nur von Interesse, eine hohe Ausbeute zu erreichen, sondern einen möglichst großen Durchsatz bei geringem technischen Aufwand zu erzielen.

Dies ist jedoch durch das in der EP 35749 vorgeschlagene Verfahren nicht möglich, weil die Reaktionsvolumina, z.B. wegen der Verwendung von Bromcyan - einer sehr flüchtigen und toxischen Substanz - nicht ohne aufwendige Sicherheitsmaßnahmen zu vergrößern sind.
Außerdem schließt sich an jeden Reaktionsschritt eine Reinigungsprozedur an, die zu großen Ausbeuteverlusten führt.
Ein weiterer Nachteil ist die Umsetzung der Base zum Hydrochlorid. Durch die in der EP 35749 vorgeschlagene Verwendung von Alkoholen bei der Ausfällung kommt es zu einem Qualitätsverlust durch allmähliche Zersetzung des Produktes.

Ein solches Verfahren ist umständlich, unwirtschaftlich und verlustreich und ist daher für eine industrielle Produktion nicht geeignet.

Es ist Aufgabe der vorliegenden Erfindung, ein technisches Verfahren bereitzustellen, das die Nachteile des geschilderten Standes der Technik nicht aufweist und insbesondere den Durchsatz großer Substanzmengen, bei verbesserter Qualität des Endproduktes, ermöglicht.

Es wurde nun überraschenderweise gefunden, daß durch Hydrierung von 6-Phthalimidomethyl-5H-dibenz[b,e]-azepin (I), anschließender alkalischer Hydrazinolyse, Reaktion mit in situ-hergestelltem Bromcyan und Ausfällen der entstandenen Base (IV) als Hydrochlorid die Synthese von 3-Amino-9,13b-dihydro-1H-dibenz[c,f]imidazo(1,5-a)azepin-hydrochlorid (V) so vorteilhaft gestaltet werden kann, daß die technische Herstellung ermöglicht wird. Außerdem ist mit dem erfindungsgemäßen Verfahren eine beträchtliche Ausbeutesteigerung verbunden.

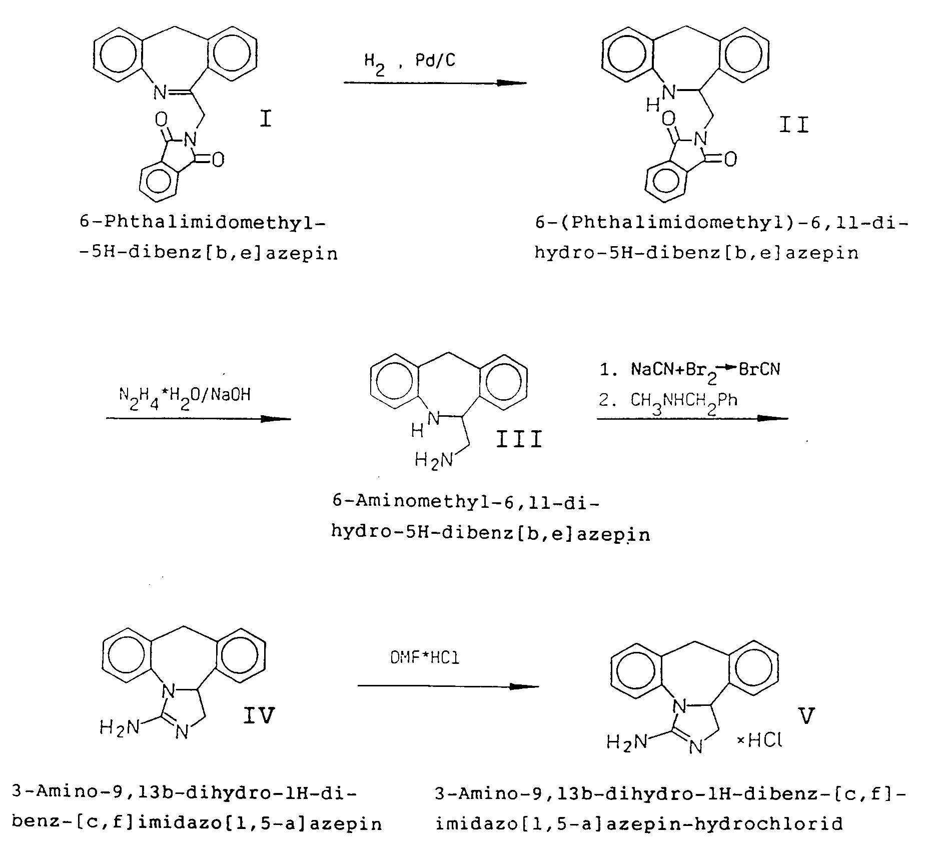

Für die Hydrierung wird 6-Phthalimidomethyl-5H-dibenz[b,e]azepin (I), das nach bekannten Methoden zugänglich ist, in Dimethylformamid (bei Verwendung von Ameisensäure) oder Dimethylacetamid (bei Verwendung von Essigsäure) mit Wasserstoff, in einem Druckbereich von 1 bis 10 bar, bevorzugt 6 bis 8 bar, besonders bevorzugt 7 bar, und unter Palladium/Kohle-Katalyse bei einer Temperatur von 40 bis 100°C, bevorzugt 60-80°C, besonders bevorzugt 70°C, umgesetzt. Dem Reaktionsgemisch sind 0,5 bis 5 mol/mol, bevorzugt 1 bis 3 mol/mol, besonders bevorzugt 2 mol/mol Essigsäure oder Ameisensäure zugefügt. Bei der Hydrierung in reinen organischen Säuren würde das Reaktionsprodukt ausfallen und müßte in aufwendiger Weise vom Katalysator abgetrennt werden.

Dieser Effekt erlaubt vorteilhafterweise den Durchsatz größerer Substanzmengen. Außerdem muß das Hydrierungsprodukt nicht durch eine zusätzliche Extraktion vom Katalysator abgetrennt werden. Nach der Hydrierungsreaktion kann der Katalysator abfiltriert und nach Abdestillation eines Teiles des Lösungsmittels das Produkt mit 90 % Ausbeute durch Zugabe von Aceton ausgefällt werden.

Nach dem erfindungsgemäßen Verfahren wird das Hydrierungsprodukt einer alkalischen Hydrazinolysereaktion unterworfen, und die Extrakte der Reaktion direkt mit in situ-hergestelltem Bromcyan umgesetzt.
Dazu wird 6-(Phthalimidomethyl)-6,11-dihydro-5H-dibenz[b,e]azepin (II) in einer alkalischen Mischung aus Wasser und einer hochsiedenden Flüssigkeit, die jedoch nicht mit chlorierten Kohlenwasserstoffen mischbar ist, zum Beispiel Ethylenglykol, Propylenglykol, Glycerin, bevorzugt Ethylenglykol, für zwei Stunden bei 100 bis 150°C, bevorzugt bei 110-120°C, mit Hydrazinhydrat umgesetzt. Als Base wird eine Alkalilauge, bevorzugt Natronlauge verwendet.

Die alkalische Hydrolyse nach einer Hydrazinolysereaktion ist bekannt (Gibson, M.S. und Bradshaw, R.W., Angew. Chem 80, 986-996(1968)), nicht jedoch die direkte Umsetzung mit Hydrazin in einer alkalischen Lösung, die auf diese Weise die direkte Extraktion des entstandenen Amins (III) ermöglicht.

Außerdem erlaubt die Hydrazinolyse unter alkalischen Bedingungen vorteilhafterweise die Verringerung des einzusetzenden Hydrazins auf stöchiometrische Mengen, was die Handhabung dieser carcinogenen Substanz beträchtlich vereinfacht.

Das erfindungsgemäße Verfahren erlaubt außerdem durch den Einsatz von mit Wasser mischbaren, hochsiedenden Flüssigkeiten, wie Glykol, eine Erhöhung der Reaktionstemperatur und damit kürzere Reaktionszeiten. Auf diese Weise gelingt ebenfalls die direkte Extraktion des sauberen Produktes mit chlorierten Kohlenwasserstoffen für die sich anschließende Reaktion mit Bromcyan. Alle Verunreinigungen, zum Beispiel das bei der Reaktion entstehende Natriumsalz des Phthalhydrazids, verbleiben in der wäßrigen Phase, so daß keine gesonderten Aufreinigungsschritte notwendig sind.

Die Extraktion erfolgt bei 15°C mit einem chlorierten Kohlenwasserstoff, der mit dem Reaktionsgemisch der Hydrazinolyse nicht mischbar ist und eine größere Dichte besitzt, zum Beispiel Chloroform, Tetrachlorkohlenstoff, 1,2-Dichlorethan, bevorzugt Dichlormethan.

Die Extraktionsphase wird zur Reaktion mit Bromcyan zu einer wäßrigen Lösung, gebildet aus einer wäßrigen Natriumbromidlösung, die bei 5 bis 25°C, bevorzugt bei 10 bis 15°C mit Brom, und anschließend bei 10 bis 15°C mit einer wäßrigen Natriumcyanidlösung umgesetzt wurde, versetzt.

Die Mischung wird bei 5 bis 35°C, bevorzugt bei 15 bis 25°C gerührt.

Dabei reagiert das in situ entstehende Bromcyan mit dem Amin. Die direkte Handhabung großer Mengen Bromcyan entfällt damit.

Nach Reaktionsende wird zur Zerstörung des überschüssigen Bromcyans eine wenig flüchtige Base zugesetzt, deren Reaktionsprodukt mit Bromcyan in Wasser oder in Benzol, Toluol, Xylol oder Halogenbenzolen löslich ist, zum Beispiel sekundäre Monoamine wie N,N-Dibenzylamin, N,N-Dibutylamin, Diethanolamin, Diisopropylamin, aber auch N-Ethylbenzylamin, Sarcosin-Na-Salz und bevorzugt N-Methylbenzylamin. Nach Zusatz der Base wird für 15 Minuten unter Rückfluß erhitzt.

Die Handhabung von Bromcyan macht im technischen Maßstab umfangreiche Sichtsheitsmaßnahmen erforderlich.
Durch die hier benutzte in situ-Herstellung ist es möglich, die Reaktion gefahrlos auch im technischen Maßstab durchzuführen und gleichzeitig das Produkt ohne komplizierte Reinigungsschritte sauber zu isolieren:
Zur selektiven Ausfällung von 3-Amino-9,13b-dihydro-1H-dibenz[c,f]imidazo[1,5-a]azepin (IV) wird das organische Lösungsmittel abdestilliert, mit Benzol oder Xylol oder einem Halogenbenzol, bevorzugt jedoch Toluol versetzt und die Base durch Zugabe von Natronlauge ausgefällt. Das entstandene Amin (IV) fällt in hoher Reinheit und mit einer Ausbeute von 76 % an.

Die in EP 35749 beschriebene Verwendung von Alkoholen wie Methanol als Lösungsmittel zur Herstellung des Hydrochlorids führt zu einer allmählichen Zersetzung der Verbindung. Überraschenderweise wurde gefunden, daß die Ausfällung des 3-Amino-9,13b-dihydro-1H-dibenz[c,f]imidazo[1,5-a]azepins aus Dimethylformamid als Hydrochlorid (V) in fast quantitativer Ausbeute und mit guter Stabilität gelingt.

Das Amin wird dazu in Dimethylformamid, das gelöstes Salzsäuregas in einem Konzentrationsbereich von 2,0 bis 7,0 Gew.-%, bevorzugt 4,7 Gew.-%, enthält, unter Erwärmung gelöst und nach Filtration und teilweisen Abdestillation des Lösungsmittels gekühlt. Dabei fällt das Hydrochlorid aus. Nach Zentrifugation, Waschen und Trocknen ergibt sich eine Ausbeute von 90 %.

Die Gesamtausbeute dieses Verfahrens liegt bei 61,6 % gegenüber 41,8 % des in der EP 35749 beschriebenen Verfahrens.

Das erfindungsgemäße Verfahren stellt damit eine wesentliche Verbesserung gegenüber dem zum Stand der Technik zählenden Verfahren dar. Dies gilt sowohl für den Durchsatz, der insbesondere durch die verbesserte Löslichkeit des Hydrierungsproduktes gesteigert werden konnte, als auch für die Verringerung der Zahl der Synthesestufen und der Reinigungsschritte. Außerdem konnte die Reaktionsausbeute deutlich gesteigert werden.

Die nachstehenden Beispiele dienen zur Erläuterung der Erfindung.

### Beispiel 1: Herstellung von 6-(Phthalimidomethyl)-6,11-dihydro-5H-dibenz[b,e]azepin (II)

In einem 1200 l VA-Treibstrahl-Schlaufenreaktor werden eine Suspension von 110.0 kg 6-Phthalimidomethyl-5H-dibenz[b,e]azepin in 911,4 l Dimethylformamid, 28,7 kg Ameisensäure und eine Aufschlämmung von 12,5 kg 10 % Palladiumkohle in 25 l Dimethylformamid eingezogen. Das Reaktionsgemisch wird auf 70°C erwärmt und bei 7 bar abs. Wasserstoffdruck hydriert. Nach Abfiltration des Katalysators und Waschen der Reaktionsapparatur und des Katalysators mit 80 l Dimethylformamid werden von den vereinigten Filtraten 960 l Dimethylformamid bei 70 bis 80°C abdestilliert. Nach Abkühlung auf 50°C werden 150 l Aceton zugegeben, nach weiterem Abkühlen auf 15°C die ausgefallenen Kristalle abzentrifugiert, mit Aceton gewaschen und getrocknet.
- Ausbeute:: 99,5 kg 6-(Phthalimidomethyl)-6,11-dihydro-5H-dibenz[b,e]azepin (90 % d. Th.)

### Beispiel 2: Herstellung von 3-Amino-9,13b-dihydro-1H-dibenz[c,f]imidazo[1,5-a]azepin (IV)

In einem 1000 l VA-Rührwerksapparat werden nacheinander 62,3 kg 6-(Phthalimidomethyl)-6,11-dihydro-5H-dibenz [b,e]azepin, 176 l Glykol, 9 kg Hydrazinhydrat und 10,8 l 45 % Natronlauge eingebracht und zwei Stunden auf 110°C erhitzt (Lösung 1).

In einem 500 l Email-Apparat werden währenddessen 19,9 kg wasserfreies Natriumbromid in 88 l Wasser gelöst und bei 10 bis 15°C 29,6 kg Brom zudosiert. Anschließend wird bei 10 bis 15°C ein Gemisch aus 34,0 kg 26,5 % Natriumcyanidlösung und 35,2 l Wasser zugegeben (Lösung 2).

Lösung 1 wird auf 15°C abgekühlt, mit 315 l Wasser versetzt und mit 140,8 l Dichlormethan extrahiert. Die Dichlormethanphase wird bei 15 bis 20°C mit Lösung 2 versetzt. Die Extraktion von Lösung 1 wird mit 95 l und mit 10 l Dichlormethan zweimal wiederholt und die Extrakte ebenfalls zu Lösung 2 gegeben. Das Zweiphasengemisch wird über Nacht bei 20 bis 25°C gerührt, anschließend werden 10,7 kg N-Methylbenzylamin zugegeben und 15 Minuten zum Rückfluß erhitzt.

Nach Abdestillation des organischen Lösungsmittels wird bei 60°C mit 88 l Toluol und 39,6 l 45 % Natronlauge versetzt, nach 30 min auf 5°C gekühlt und 30 min gerührt. Der Niederschlag wird abzentrifugiert, gewaschen und getrocknet.
- Ausbeute:: 33,3 kg 3-Amino-9,13b-dihydro-1H-dibenz[c,f]imidazo[1,5-a]azepin (76 % d. Th.)

### Beispiel 3: Herstellung von 3-Amino-9,13b-dihydro-1H-dibenz[c,f]imidazo[1,5-a]azepin-hydrochlorid (V)

In einem 250 l Email-Apparat werden 60 kg 6-(Phthalimidomethyl)-6,11-dihydro-5H-dibenz[b,e]azepin, 157,9 l Dimethylformamid und 48,4 kg einer 20 % Salzsäuregas-Lösung in Dimethylformamid eingebracht. Nach Erhitzen auf 120°C wird die Lösung filtriert und nach Waschen der Apparatur mit 23,6 l Dimethylformamid werden 75 bis 80 l Lösungsmittel abdestilliert und der Inhalt des Reaktionsapparates auf 5°C abgekühlt. Die angefallenen Kristalle werden abzentrifugiert, gewaschen und getrocknet.
- Ausbeute:: 61,9 kg 3-Amino-9,13b-dihydro-1H-dibenz[c,f]imidazo[1,5-a]azepin-hydrochlorid (90 % d. Th.)

## Patentansprüche

1. Verfahren zur Herstellung von 3-Amino-9,13b-dihydro-1H-dibenz[c,f]imidazo [1,5-a]azepin-hydrochlorid, dadurch gekennzeichnet, daß man 6-Phthalimidomethyl-5H-dibenz[b,e]azepin in Dimethylformamid unter Zusatz von Ameisensäure oder in Dimethylacetamid unter Zusatz von Essigsäure, wobei das Molverhältnis der zu hydrierenden Verbindung zu der jeweiligen Säure 1 : 0,5 bis 1 : 5 beträgt, mit Wasserstoff in Gegenwart von Palladium/Kohle als Katalysator bei 1 bis 10 bar Wasserstoffdruck und bei einer Temperatur von 40 bis 100°C hydriert, das Hydrierungsprodukt in alkalischer Lösung unter Zusatz eines höhersiedenden, wassermischbaren Lösungsmittels bei 100 bis 150°C einer Hydrazinolyse unterwirft, zur Abtrennung des Hydrazinolyseprodukts mit einem chlorierten Kohlenwasserstoff extrahiert, den Extrakt mit in situ hergestelltem Bromcyan bei 5 bis 35 °C umsetzt und nach der Zerstörung des überschüssigen Bromcyans durch Zugabe einer wenig flüchtigen Base und dem Abdestillieren des organischen Lösungsmittels schließlich das 3-Amino-azepin nach Umsetzung mit Chlorwasserstoff als Hydrochlorid isoliert.

2. Hydrierung von 6-Phthalimidomethyl-5H-dibenz[b,e]azepin gemäß Anspruch 1, dadurch gekennzeichnet, daß die Hydrierung in Dimethylformamid unter Zusatz von Ameisensäure in einem Konzentrationsbereich von 1 bis 3 mol Säure pro mol des Azepins, bevorzugt 2 mol/mol mit Palladium/Kohle als Katalysator bei 6-8 bar, bevorzugt 7 bar Wasserstoffdruck und bei einer Temperatur von 60-80°C, bevorzugt 70°C, durchgeführt wird.

3. Hydrazinolyse von 6-(Phthalimidomethyl)-6,11-dihydro-5H-dibenz[b,e]azepin gemäß Anspruch 1, dadurch gekennzeichnet, daß die Hydrazinolyse in einer alkalischen Mischung aus Wasser und einer hochsiedenden Flüssigkeit, die nicht mit chlorierten Kohlenwasserstoffen mischbar ist, erfolgt.

4. Hydrazinolyse von 6-(Phthalimidomethyl)-6,11-dihydro-5H-dibenz[b,e]azepin gemäß Anspruch 1, dadurch gekennzeichnet, daß
a) mit Hydrazinhydrat in alkalischer Lösung zwischen 100 und 150°C, bevorzugt bei 110-120°C, umgesetzt wird,
b) als Base eine Alkalilauge, bevorzugt Natronlauge, eingesetzt wird,
c) in höhersiedenden, wassermischbaren Lösungsmitteln, bevorzugt Ethylenglykol, Propylenglykol, Glycerin, besonders bevorzugt Ethylenglykol, gearbeitet wird, und
d) zur Isolierung des Hydrazinolyseproduktes mit einem chlorierten Kohlenwasserstoff extrahiert wird, bevorzugt Chloroform, Tetrachlorkohlenstoff, 1,2-Dichlorethan, besonders bevorzugt Dichlormethan.

5. Umsetzung von 6-Aminomethyl-6,11-dihydro-5H-dibenz[b,e]azepin mit Bromcyan gemäß Anspruch 1, dadurch gekennzeichnet, daß
a) Natriumbromid in wäßriger Lösung bei 5 bis 25°C, bevorzugt bei 10 bis 15° C mit Brom und anschließend bei 10 bis 15°C mit Natriumcyanid umgesetzt und
b) diese Lösung bei 5 bis 35°C, bevorzugt bei 15 bis 25°C, mit der Extraktionsphase, die das Hydrazinolyseprodukt enthält, gerührt und anschließend
c) eine Base zugesetzt wird, bevorzugt N,N-Dibenzylamin, N,N-Dibutylamin, Diethanolamin, Diisopropylamin, N-Ethylbenzylamin, Sarcosin-Na-Salz und besonders bevorzugt N-Methylbenzylamin und
d) das entstandene 3-Amino-9,13b-dihydro-1H-dibenz[c,f]imidazo[1,5-a]azepin in Benzol, Xylol oder einem Halogenbenzol, bevorzugt Toluol aufgenommen und durch Zugabe von Natronlauge ausgefällt wird.

6. Herstellung des Hydrochlorids gemäß Anspruch 1, dadurch gekennzeichnet, daß die Base in Dimethylformamid, das gelöstes Salzsäuregas in einem Konzentrationsbereich von 2,0 bis 7,0 Gew.-%, bevorzugt 4,7 Gew.-% enthält, unter Erwärmung gelöst und nach Filtration und Abdestillation von ca. einem Drittel des Lösungsmittels gekühlt wird, wobei 3-Amino-9,13b-dihydro-1H-dibenz[c,f]imidazo[1,5-a]azepin-hydrochlorid ausfällt.

## Claims

1. Process for preparing 3-amino-9,13b-dihydro-1H-dibenz[c,f]imidazo[1,5-a]azepine-hydrochloride, characterised in that 6-phthalimidomethyl-5H-dibenz[b,e]azepine is hydrogenated in dimethylformamide with the addition of formic acid or in dimethylacetamide with the addition of acetic acid, the molar ratio of the compound which is to be hydrogenated to the particular acid being 1 : 0.5 to 1 : 5, with hydrogen in the presence of palladium/charcoal as catalyst at a hydrogen pressure of 1 to 10 bar and at a temperature of 40 to 100°C, the hydrogenation product is subjected to hydrazinolysis in alkaline solution with the addition of a higher boiling, water-miscible solvent at 100 to 150°C, extracted with a chlorinated hydrocarbon in order to separate off the hydrazinolysis product, the extract is reacted with bromocyanogen prepared in situ at 5 to 35°C and after destruction of the excess bromocyanogen by the addition of a less volatile base and after the organic solvent has been distilled off the 3-amino-azepine is finally isolated as the hydrochloride after reaction with hydrogen chloride.

2. Hydrogenation of 6-phthalimidomethyl-5H-dibenz[b,e]azepine according to claim 1, characterised in that the hydrogenation is carried out in dimethylformamide with the addition of formic acid in a concentration range of from 1 to 3 mol of acid per mol of azepine, preferably 2 mol/mol, using palladium/charcoal as catalyst at 6 to 8 bar, preferably 7 bar of hydrogen pressure and at a temperature of 60 to 80°C, preferably 70°C.

3. Hydrazinolysis of 6-(phthalimidomethyl)-6,11-dihydro-5H-dibenz[b,e]azepine according to claim 1, characterised in that the hydrazinolysis is carried out in an alkaline mixture of water and a high-boiling liquid which is immiscible with chlorinated hydrocarbons.

4. Hydrazinolysis of 6-(phthalimidomethyl)-6,11-dihydro-5H-dibenz[b,e]azepine according to claim 1, characterised in that
a) the reaction is carried out with hydrazine hydrate in an alkaline solution at between 100 and 150°C, preferably at 110 to 120°C,
b) the base used is an alkali metal hydroxide solution, preferably sodium hydroxide solution,
c) the work is done in higher-boiling water-miscible solvents, preferably ethylene glycol, propylene glycol, glycerol, most preferably ethylene glycol and
d) in order to isolate the hydrazinolysis product, extraction is carried out with a chlorinated hydrocarbon, preferably chloroform, carbon tetrachloride, 1,2-dichloroethane, most preferably dichloromethane.

5. Reaction of 6-aminomethyl-6,11-dihydro-5H-dibenz[b,e]azepine with bromocyanogen according to claim 1, characterised in that
a) sodium bromide in aqueous solution is reacted with bromine at 5 to 25°C, preferably 10 to 15°C, and then with sodium cyanide at 10 to 15°C and
b) this solution is stirred at 5 to 35°C, preferably at 15 to 25°C, with the extraction phase which contains the hydrazinolysis product and then
c) a base is added, preferably N,N-dibenzylamine, N,N-dibutylamine, diethanolamine, diisopropylamine, N-ethylbenzylamine, sarcosine Na salt and especially N-methylbenzylamine and
d) the resulting 3-amino-9,13b-dihydro-1H-dibenz[c,f]imidazo[1,5-a]azepine is taken up in benzene, xylene or a halobensene, preferably toluene, and precipitated by the addition of sodium hydroxide solution.

6. Preparation of the hydrochloride according to claim 1, characterised in that the base is dissolved, with heating, in dimethylformamide which contains dissolved hydrochloric acid gas in a concentration range from 2.0 to 7.0 wt.-%, preferably 4.7 wt.-%, and after filtration and distillation of about one third of the solvent, the mixture is cooled, whereupon 3-amino-9,13b-dihydro-1H-dibenz[c,f]imidazo[1,5-a]azepine-hydrochloride is precipitated.

## Revendications

1. Procédé de préparation du chlorhydrate de la 3-amino-9,13b-dihydro-1H-dibenzo[c,f]imidazo[1,5-a]azépine, caractérisé en ce que l'on hydrogène la 6-phtalimidométhyl-5H-dibenzo[b,e]azépine dans du diméthylformamide en ajoutant de l'acide formique, ou dans du diméthylacétamide en ajoutant de l'acide acétique, le rapport molaire du composé à hydrogéner à l'acide correspondant étant de 1:0,5 à 1:5, avec de l'hydrogène en présence de palladium/charbon comme catalyseur, sous une pression d'hydrogène de 1 à 10 bars et à une température de 40 à 100°C, on soumet le produit de l'hydrogénation à une hydrazinolyse en solution basique en ajoutant un solvant de point d'ébullition élevé miscible à l'eau, à une température de 100 à 150°C, on extrait avec un hydrocarbure chloré pour séparer le produit de l'hydrazinolyse, on fait réagir l'extrait à une température de 5 à 35°C avec du bromure de cyanogène préparé *in situ,* et, après avoir détruit le bromure de cyanogène en excès par addition d'une base peu volatile et distillé le solvant organique, on isole enfin la 3-aminoazépine sous forme de son chlorhydrate, après une réaction avec du chlorure d'hydrogène.

2. Hydrogénation de la 6-phtalimidométhyl-5H-dibenzo[b,e]azépine selon la revendication 1, caractérisée en ce que l'on effectue l'hydrogénation dans du diméthylformamide en ajoutant de l'acide formique à une concentration comprise entre 1 et 3 moles d'acide par mole d'azépine, de préférence de 2 moles/mole, avec du palladium/charbon comme catalyseur, sous une pression d'hydrogène de 6 à 8 bars, de préférence de 7 bars, et à une température de 60 à 80°C, de préférence de 70°C.

3. Hydrazinolyse de la 6-phtalimidométhyl-6,11-dihydro-5H-dibenzo[b,e]azépine selon la revendication 1, caractérisée en ce que l'on réalise l'hydrazinolyse dans un mélange basique d'eau et d'un liquide de point d'ébullition élevé qui n'est pas miscible aux hydrocarbures chlorés.

4. Hydrazinolyse de la 6-phtalimidométhyl-6,11-dihydro-5H-dibenzo[b,e]azépine selon la revendication 1, caractérisée en ce que
a) on la fait réagir avec de l'hydrate d'hydrazine en solution basique à une température comprise entre 100 et 150°C, de préférence entre 110 et 120°C,
b) on utilise comme base une solution alcaline, de préférence une solution de soude,
c) on travaille dans des solvants miscibles à l'eau, de point d'ébullition élevé, de préférence dans l'éthylèneglycol, le propylèneglycol, le glycérol, de façon particulièrement préférée dans l'éthylèneglycol, et
d) pour isoler le produit de l'hydrazinolyse, on extrait avec un hydrocarbure chloré, de préférence le chloroforme, le tétrachlorure de carbone, le 1,2-dichloréthane, de façon particulièrement préférée le dichlorométhane.

5. Réaction de la 6-aminométhyl-6,11-dihydro-5H-dibenzo[b,e]azépine avec du bromure de cyanogène selon la revendication 1, caractérisée en ce que
a) on fait réagir du bromure de sodium en solution aqueuse avec du brome à une température de 5 à 25°C, de préférence de 10 à 15°C, puis avec du cyanure de sodium à une température de 10 à 15°C, et
b) on agite cette solution à une température de 5 à 35°C, de préférence de 15 à 25°C, avec la phase d'extraction qui contient le produit de l'hydrazinolyse, puis
c) on ajoute une base, de préférence la N,N-dibenzylamine, la N,N-dibutylamine, la diéthanolamine, le diisopropylamine, la N-éthylbenzylamine, le sel de Na de la sarcosine, et de façon particulièrement préférée la N-méthylbenzylamine, et
d) on reprend la 3-amino-9,13b-dihydro-1H-dibenzo[c,f]imidazo[1,5-a]azépine dans du benzène, du xylène ou un halogénobenzène, de préférence dans du toluène, et on la précipite en ajoutant une solution de soude.

6. Préparation du chlorhydrate selon la revendication 1, caractérisée en ce que l'on dissout en chauffant la base dans du diméthylformamide qui contient le gaz chlorhydrique dissous à une concentration comprise entre 2,0 et 7,0 % en masse, de préférence à une concentration de 4,7 % en masse, et, après une filtration et une séparation par distillation d'environ un tiers du solvant, on refroidit, ce qui entraîne la précipitation du chlorhydrate de la 3-amino-9,13b-dihydro-1H-dibenzo[c,f]imidazo[1,5-a]azépine.
